# EUROPEAN PATENT SPECIFICATION

(11) **EP 2 081 907 B1**
(45) Date of publication and mention of the grant of the patent: **26.01.2011**
(21) Application number: 07821255.2
(22) Date of filing: 12.10.2007
(51) Int. Cl.: C07D 215/48, A61K 31/4709, A61P 25/18

(54) **ARYL PIPERAZINE DERIVATIVES USEFUL FOR THE TREATMENT OF NEUROPSYCHIATRY DISORDERS**
FÜR DIE BEHANDLUNG VON NEUROPSYCHIATRISCHEN STÖRUNGEN GEEIGNETE ARYLPIPERAZINDERIVATE
DERIVES D'ARYLPIPERAZINE UTILES POUR LE TRAITEMENT DE TROUBLES NEUROPSYCHIATRIQUES

(30) Priority: 13.10.2006 DK 200601328; 13.10.2006 US 851310 P
(43) Date of publication of application: 29.07.2009
(73) Proprietor: Universita Degli Studi di Siena, 53100 Siena (IT)
(72) Inventor: CAMPIANI, Giuseppe, 53042 Chianciano Terme (siena) (IT); BUTINI, Stefania, 53100 Siena (IT); FATTORUSSO, Caterina, 80128 Napoli (IT); FRANCESCHINI, Silvia, 58033 Castel del Piano (gr) (IT); THALE, Zia Irene, 2720 Vanløse (DK); NIELSEN, Karin Sandager, 2750 Ballerup (DK); SCHEEL-KRÜGER, Jørgen, 2600 Glostrup (DK); MADSEN, Lars Siim, 2750 Ballerup (DK)
(74) Representative: Madsen, Inger Margrethe Schelde
(86) International application number: PCT/EP2007/060888
(87) International publication number: WO 2008/043839

(56) References cited:
- WO-A-03/028728
- WO-A-2006/072608
- US-A- 4 803 203
- LEOPOLDO MARCELLO ET AL: "Design, synthesis, and binding affinities of potential positron emission tomography (PET) ligands for visualization of brain dopamine D-3 receptors" JOURNAL OF MEDICINAL CHEMISTRY, vol. 49, no. 1, January 2006 (2006-01), pages 358-365, XP002465112 ISSN: 0022-2623

## Description

### TECHNICAL FIELD

This invention provides novel aryl piperazine derivatives having medical utility, in particular as modulators of dopamine and serotonin receptors, preferably the D₃, 5HT_{1A} and 5-HT_{2A} receptor subtypes, and in particular useful for the treatment of neuropsychiatric disorders, incl. schizophrenia.

### BACKGROUND ART

Dopamine is involved in several important functions, excitatory and inhibitory, via dopaminergic receptors in the central and peripherical nervous system. Dopamine receptors were originally classified into two main groups: D₁ and D₂. The five currently cloned dopamine receptors fall into these classes. Thus, the D₁-like receptors include D₁ and D₅, while the D₂-like receptors include D₂, D₃ and D₄.

The dopamine receptors, and in particular the D₂-like receptors, are recognised as potential therapeutic targets for various neurological and psychiatric disorders, in particular psychotic disorders, incl. schizophrenia. Other therapeutic indications associated with the dopamine receptors include depression, Parkinson's disease, Huntington's disease, movement disorders such as dystonia, anxiety, restlessness, obsessive-compulsive disorders, mania, geriatric disorders, dementia, sexual dysfunction, musculo-skeletal pain symptoms, e.g. pain associated with fibromyalgia, substance abuse (cocaine abuse and addiction), abuse liability and withdrawal symptoms in drug addicts, and sleep disorders.

Still other therapeutic indications include eating disorders such as overeating, compulsive overeating, inability to regulate eating, bulimia and Binge-eating disorder.

Also the compounds of the invention may be useful for the treatment of abuse liability and withdrawal symptoms caused by termination of use of addictive substances. Such addictive substances include nicotine containing products such as tobacco, opioids such as heroin, cocaine and morphine, cannabis, benzodiazepines, benzodiazepine-like drugs, and alcohol. Withdrawal from addictive substances is in general a traumatic experience characterised by anxiety and frustration, anger, anxiety, difficulties in concentrating, restlessness, decreased heart rate and increased appetite and weight gain.

Finally receptor selective ligands find use as diagnostic tools in diagnostic methods, and in particular for *in vivo* receptor imaging (neuroimaging).

WO 2006/072608 describes aryl piperazine derivatives useful as as modulators of dopamine and serotonin receptors. However, the aryl piperazine derivatives of the present invention have not been reported.

WO 03/028728 describes certain substituted piperazinyl-butyl-carboxamides, incl. 3- and 4-quinoline derivatives, useful as dopamine D3 selective ligands. However, the 6-quinoline derivatives of the present invention have not been disclosed.

Leopoldo et al.; J. Med. Chem. 2006 49 358-365, describe the design, synthesis, and binding affinities of potential PET ligands, incl. certain 6-quinoline derivatives, for visualization of brain dopamine D3 receptors. However, the 6-quinoline derivatives of the present invention have not been disclosed.

US 4,803,203 discloses certain heterocyclic piperazinyl alkoxy-benzheterocyclic derivatives useful as antipsychotic agents. However, the 6-quinoline derivatives of the present invention have not been disclosed.

### SUMMARY OF THE INVENTION

According to the present invention it has now been found that a particular group of aryl piperazine derivatives show superior activity as modulators of dopamine and serotonin receptors, preferably the D₃, 5HT_{1A} and 5-HT_{2A} receptor subtypes, has no significant activity on hERG, and has a good bioavailability when administered p.o.

Therefore, in its first aspect, the invention provides novel aryl piperazine derivatives represented by Formula I 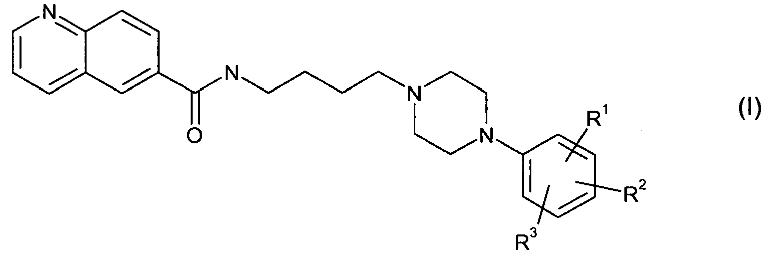 an enantiomer thereof or a mixture of its enantiomers, or a pharmaceutically acceptable salt thereof, wherein,
R¹, R² and R³, independently of each other, represent hydrogen, methyl, hydroxy, methoxy, halo, trifluoromethyl, cyano or carboxy.

In another aspect the invention relates to the use of the aryl piperazine derivative of the invention, or a pharmaceutically acceptable salt thereof, for the manufacture of a pharmaceutical composition.

Viewed from yet another aspect the invention relates to the use of the aryl piperazine derivative of the invention, or a pharmaceutically acceptable salt thereof, for use as a medicament, or for the manufacture of a pharmaceutical composition for the treatment, prevention or alleviation of a disease or a disorder or a condition of a mammal, including a human, which disease, disorder or condition is responsive to modulation of the dopamine and serotonin receptors.

Other objects of the invention will be apparent to the person skilled in the art from the following detailed description and examples.

### DETAILED DISCLOSURE OF THE INVENTION

According to the present invention it has now been found that a particular group of aryl piperazine derivatives show a superior biological profile as modulators of dopamine and serotonin receptors.

Therefore, in its first aspect, the invention provides novel aryl piperazine derivatives represented by Formula I 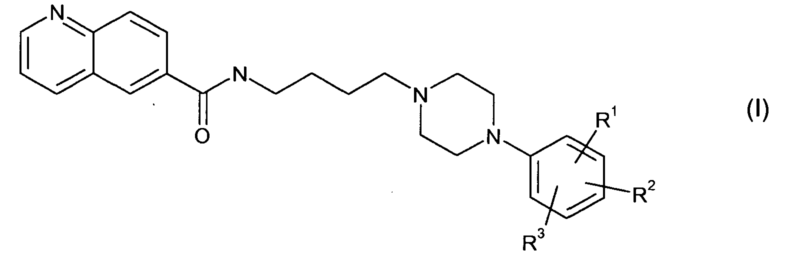 an enantiomer thereof or a mixture of its enantiomers, or a pharmaceutically acceptable salt thereof, wherein, R¹, R² and R³, independently of each other, represent hydrogen, methyl, hydroxy, methoxy, halo, trifluoromethyl, cyano or carboxy.

In a preferred embodiment the aryl piperazine derivative of the invention is a compound of Formula I, wherein R¹, R² and R³, independently of each other, represent hydrogen, methyl, methoxy, halo, trifluoromethyl, cyano or carboxy.

In another preferred embodiment the aryl piperazine derivative of the invention is a compound of Formula I, wherein R¹, R² and R³, independently of each other, represent hydrogen, methyl, hydroxy, methoxy, halo or trifluoromethyl.

In a more preferred embodiment R¹, R² and R³, independently of each other, represent hydrogen, methyl, methoxy, halo or trifluoromethyl.

In an even more preferred embodiment R¹, R² and R³, independently of each other, represent hydrogen, hydroxy or halo.

In a still more preferred embodiment R¹, R² and R³, independently of each other, represent hydrogen or halo.

In a third preferred embodiment the aryl piperazine derivative of the invention is a compound of Formula I, wherein R¹, R² and R³, independently of each other, represent hydrogen, halo, hydroxy or trifluoromethyl.

In a more preferred embodiment R¹, R² and R³, independently of each other, represent hydrogen, halo or trifluoromethyl.

In an even more preferred embodiment R¹, R² and R³, independently of each other, represent hydrogen or halo.

In a fourth preferred embodiment the aryl piperazine derivative of the invention is a compound of Formula I, wherein R¹, R² and R³, independently of each other, represent hydrogen, fluoro, chloro, bromo or trifluoromethyl.

In a more preferred embodiment R¹, R² and R³, independently of each other, represent hydrogen or fluoro.

In a fifth preferred embodiment the aryl piperazine derivative of the invention is a compound of Formula I, wherein one of R¹, R² and R³, represents hydrogen or hydroxy; and the two others of R¹, R² and R³, independently of each other, represent methyl, methoxy, halo, trifluoromethyl, cyano or carboxy.

In a more preferred embodiment one of R¹, R² and R³, represents hydrogen; and the two others of R¹, R² and R³ represent methyl, methoxy, halo, trifluoromethyl, cyano or carboxy.

In an even more preferred embodiment one of R¹, R² and R³, represents hydrogen or hydroxy; and the two others of R¹, R² and R³ represent halo, and in particular fluoro.

In a still more preferred embodiment one of R¹, R² and R³, represents hydrogen or hydroxy; and the two others of R¹, R² and R³ represent halo, and in particular fluoro.

In a yet more preferred embodiment one of R¹, R² and R³, represents hydrogen; and the two others of R¹, R² and R³ represent halo, and in particular fluoro.

In a sixth preferred embodiment the aryl piperazine derivative of the invention is a compound of Formula I, wherein two of R¹, R² and R³, represents hydrogen; and the last one of R¹, R² and R³ represent methyl, hydroxy, methoxy, halo, trifluoromethyl, cyano or carboxy.

In a more preferred embodiment two of R¹, R² and R³, represents hydrogen; and the last one of R¹, R² and R³ represent methyl, methoxy, halo, trifluoromethyl, cyano or carboxy.

In an even more preferred embodiment two of R¹, R² and R³, represents hydrogen; and the last one of R¹, R² and R³ represent hydroxy.

In a most preferred embodiment the aryl piperazine derivative of the invention is
*N*-(4-(4-Phenylpiperazin-1-yl)butyl)quinoline-6-carboxamide; or
Quinoline-6-carboxylic acid {4-[4-(2,3-difluoro-phenyl)-piperazin-1-yl]-butyl}-amide;
an enantiomer thereof or a mixture of its enantiomers, or a pharmaceutically acceptable salt thereof.

Any combination of two or more of the embodiments described herein is considered within the scope of the present invention.

### Pharmaceutically Acceptable Salts

The aryl piperazine derivatives of the invention may be provided in any form suitable for the intended administration. Suitable forms include pharmaceutically (i.e. physiologically) acceptable salts of the aryl piperazine derivatives of the invention.

Examples of pharmaceutically acceptable salts include, without limitation, the non-toxic inorganic and organic acid salts such as the hydrochloride, the hydrobromide, the nitrate, the perchlorate, the phosphate, the sulphate, the formate, the acetate, the aconate, the ascorbate, the benzenesulphonate, the benzoate, the cinnamate, the citrate, the embonate, the enantate, the fumarate, the glutamate, the glycolate, the lactate, the maleate, the malonate, the mandelate, the methanesulphonate, the naphthalene-2-sulphonate derived, the phthalate, the salicylate, the sorbate, the stearate, the succinate, the tartrate, the toluene-p-sulphonate, and the like. Such salts may be formed by procedures well known and described in the art.

### Steric Isomers

Some of the aryl piperazine derivatives of the present invention may exist in (+) and (-) forms as well as in racemic forms (±). The racemates of these isomers and the individual isomers themselves are within the scope of the present invention.

Racemic forms can be resolved into the optical antipodes by known methods and techniques. One way of separating the diastereomeric salts is by use of an optically active acid, and liberating the optically active amine compound by treatment with a base. Another method for resolving racemates into the optical antipodes is based upon chromatography on an optical active matrix. A stereoselective synthetic approach may be pursued. Racemic compounds of the present invention can thus be resolved into their optical antipodes, e.g., by fractional crystallisation of D- or L- (tartrates, mandelates, or camphorsulphonate) salts for example.

Starting materials and/or intermediate compounds used for producing the chemical compounds of the present invention may also be resolved by the formation of diastereomeric amides by reaction of the aryl piperazine derivative of the present invention with an optically active activated carboxylic acid such as that derived from (+) or (-) phenylalanine, (+) or (-) phenylglycine, (+) or (-) camphanic acid or by the formation of diastereomeric carbamates by reaction of the starting material or intermediate compound for use according to the present invention with an optically active chloroformate or the like.

Additional methods for the resolving the optical isomers are known in the art. Such methods include those described by Jaques J, Collet A, & Wilen S in "Enantiomers, Racemates, and Resolutions", John Wiley and Sons, New York (1981).

Optical active compounds can also be prepared from optical active starting materials.

### Methods of Preparation

The aryl piperazine derivatives of the invention may be prepared by conventional methods for chemical synthesis, e.g. those described in the working examples.

Generally amides may be prepared by transforming acids or acid chlorides into the corresponding hydroxy amides by a standard procedure. Esters may be obtained by reacting acidic starting materials with 1,4-dihydroxybutane. After substitution of the terminal hydroxy group by bromine, hydroxyl amides may be treated with the aryl piperazine in the presence of a base to give the desired end product. Compounds based on a ethereal tether may be synthesized starting from the appropriate phenol, which is then condensed with 14-dihydroxybutane or 1,5-dihydroxypentane, followed by transformation into the final products as described above.

Intermediate compounds invention may be resolved by the formation of diastereomeric amides by reaction with an optically active activated carboxylic acid such as that derived from (+) or (-) phenylalanine, (+) or (-) phenylglycine, (+) or (-) camphanic acid or by the formation of diastereomeric carbamates by reaction of the intermediate compound with an optically active chloroformate or the like.

### Biological Activity

The aryl piperazine derivatives of the invention were found to possess selectivity for the dopamine and serotonin receptors. Therefore, in a preferred embodiment, the invention relates to use of the aryl piperazine derivatives of the invention for the manufacture of a pharmaceutical composition for the treatment, prevention or alleviation of a disease or a disorder or a condition of a mammal, including a human, which disease, disorder or condition is responsive to modulation of the dopamine and serotonin receptors, in particular the D₃, 5HT_{1A} and 5-HT_{2A} receptor subtypes.

Moreover, the aryl piperazine derivatives of the invention has no significant activity on hERG, and has a good bioavailability when administered p.o.

Therefore, in a preferred embodiment, the invention relates to use of the aryl piperazine derivatives of the invention for the manufacture of a pharmaceutical composition for the treatment, prevention or alleviation of a disease or a disorder or a condition of a mammal, including a human, which disease, disorder or condition is responsive to modulation of the dopamine and serotonin receptors.

In a more preferred embodiment the disease, disorder or condition is a neurological or psychiatric disorders, in particular psychotic disorders, incl. schizophrenia, depression, Parkinson's disease, Huntington's disease, movement disorders, in particular dystonia, anxiety, restlessness, obsessive-compulsive disorders, mania, geriatric disorders, dementia, sexual dysfunction, musculo-skeletal pain symptoms, in particular pain associated with fibromyalgia, sleep disorders, substance abuse or addiction, and abuse liability and withdrawal symptoms in drug addicts, cocaine abuse or addiction.

In an even more preferred embodiment the disease, disorder or condition is a neurological or psychiatric disorder, in particular a psychotic disorder, preferably schizophrenia.

In another preferred embodiment the disease, disorder or condition contemplated according to the invention is schizophrenia or Parkinson's disease.

In a third preferred embodiment the disease, disorder or condition contemplated according to the invention an eating disorder, overeating, compulsive overeating, inability to regulate eating, bulimia or Binge-eating disorder.

In a fourth preferred embodiment the disease, disorder or condition contemplated according to the invention is abuse liability or withdrawal symptoms caused by termination of use of addictive substances. Such addictive substances include nicotine containing products such as tobacco, opioids such as heroin, cocaine and morphine, cannabis, benzodiazepines, benzodiazepine-like drugs, and alcohol. Withdrawal from addictive substances is in general a traumatic experience characterised by anxiety and frustration, anger, anxiety, difficulties in concentrating, restlessness, decreased heart rate and increased appetite and weight gain.

In yet another preferred embodiment the aryl piperazine derivatives of the invention are used as diagnostic tools in diagnostic methods, and in particular for *in vivo* receptor imaging (neuroimaging).

### Pharmaceutical Compositions

In another aspect the invention provides novel pharmaceutical compositions comprising a therapeutically effective amount of the aryl piperazine derivative of the invention.

While an aryl piperazine derivative of the invention for use in therapy may be administered in the form of the raw chemical compound, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

In a preferred embodiment, the invention provides pharmaceutical compositions comprising the aryl piperazine derivative of the invention, or a pharmaceutically acceptable salt or derivative thereof, together with one or more pharmaceutically acceptable carriers, and, optionally, other therapeutic and/or prophylactic ingredients, know and used in the art. The carrier(s) must be "acceptable" in the sense of being compatible with the other ingredients of the formulation and not harmful to the recipient thereof.

The pharmaceutical composition of the invention may be administered by any convenient route, which suits the desired therapy. Preferred routes of administration include oral administration, in particular in tablet, in capsule, in dragé, in powder, or in liquid form, and parenteral administration, in particular cutaneous, subcutaneous, intramuscular, or intravenous injection. The pharmaceutical composition of the invention can be prepared by any person skilled in the art, by use of standard methods and conventional techniques, appropriate to the desired formulation. When desired, compositions adapted to give sustained release of the active ingredient may be employed.

Further details on techniques for formulation and administration may be found in the latest edition of Remington's Pharmaceutical Sciences (Maack Publishing Co., Easton, PA).

The actual dosage depends on the nature and severity of the disease being treated, and is within the discretion of the physician, and may be varied by titration of the dosage to the particular circumstances of this invention to produce the desired therapeutic effect. However, it is presently contemplated that pharmaceutical compositions containing of from about 0.1 to about 500 mg of active ingredient per individual dose, preferably of from about 1 to about 100 mg, most preferred of from about 1 to about 10 mg, are suitable for therapeutic treatments.

The active ingredient may be administered in one or several doses per day. A satisfactory result can, in certain instances, be obtained at a dosage as low as 0.1 µg/kg i.v. and 1 µg/kg p.o. The upper limit of the dosage range is presently considered to be about 10 mg/kg i.v. and 100 mg/kg p.o. Preferred ranges are from about 0.1 µg/kg to about 10 mg/kg/day i.v., and from about 1 µg/kg to about 100 mg/kg/day p.o.

### EXAMPLES

The invention is further illustrated with reference to the following examples, which are not intended to be in any way limiting to the scope of the invention as claimed.

### Example 1

### Preparatory Example

The synthetic strategy followed to obtain Compounds **5₁** and **5₂** is reported in Scheme 1, below.

Commercially available 6-methylquinoline was oxidized to the corresponding quinoline-6-carboxylic acid **(2)** by using chromium trioxide in acidic medium. The acid **2** was transformed, by means of a coupling reaction with 4-aminobutanol, in the presence of 1-hydroxybenzotriazole (HOBt) and 1,3-dicyclohexylcarbodiimide (DCC), into the hydroxyl amide **3.** This latter, after bromination of the hydroxyl group, gave the bromo-derivative **4** that was treated with the opportune arylpiperazine in the presence of a base to give the desired products (**5_{1,2}**).

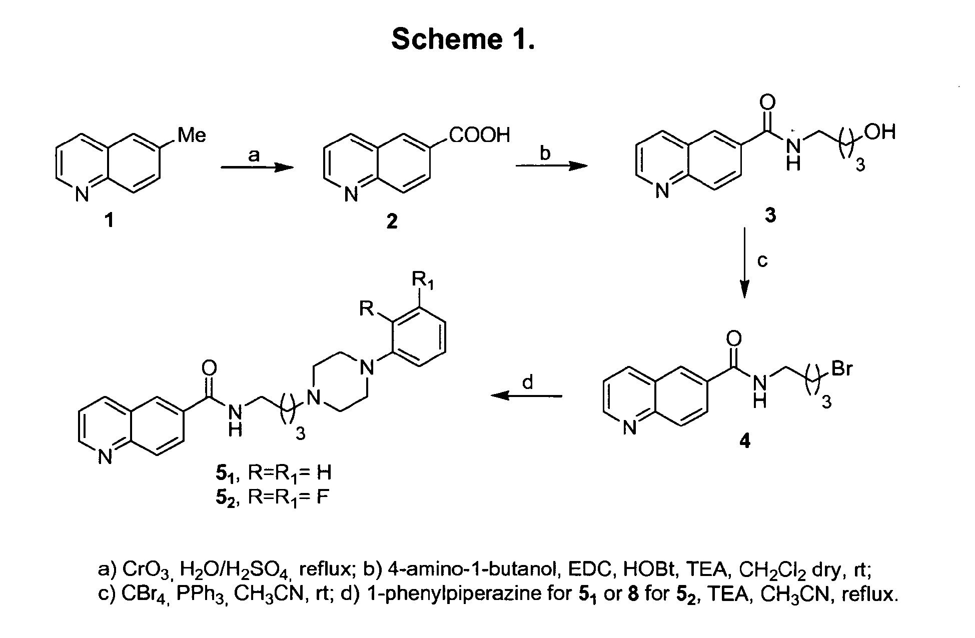

2,3-Difluoro phenylpiperazine, necessary for the synthesis of **5₂**, was obtained, according to **Scheme 2,** from Boc-piperazine and 1-bromo-2,3-difluorobenzene **(6)** by a standard palladium catalyzed reaction followed by deprotection with trifluoroacetic acid (TFA).

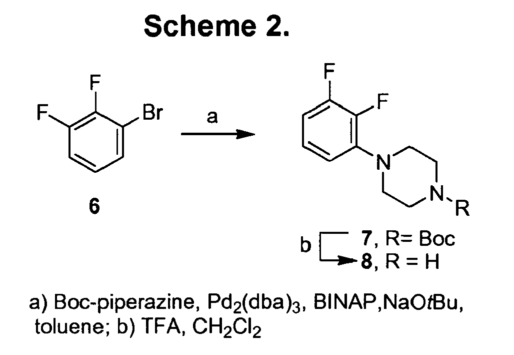

### Experimental Section

Reagents were purchased from Aldrich and were used as received. Reaction progress was monitored by TLC using Merk silica gel 60 F254 (0.040-0.063 mm) with detection by UV. Merk silica gel 60 F254 (0.040-0.063 mm) was used for column chromatography.

Melting points were determined in Pyrex capillary tubes using an Electrothermal 8103 apparatus and are uncorrected. ¹H NMR and ¹³C NMR were recorded on Varian 300 MHz spectrometer with TMS as internal standard. Splitting patterns are described as singlet (s), doublet (d), triplet (t), quartet (q), double doublet (dd) and broad (br); the value of chemical shifts (δ) are given in ppm and coupling constants (*J*) in Hertz.

ESI-MS spectra were performed by Agilent 1100 Series LC/MSD spectrometer and by LCQDeca-THERMOFINNIGAN spectrometer.

Elemental analyses were performed on a Perkin Elmer 240C elemental analyser and the results were within ± 0.4% of the theoretical values, unless otherwise noted.

Yields refer to purified products and are not optimised. All moisture-sensitive reactions were performed under argon atmosphere using oven-dried glassware and anhydrous solvents. All the organic layers were dried using anhydrous sodium sulphate.

For testing, compound **5_{1,2}** was transformed into the corresponding hydrochloride salt by a standard procedure.

### 6-Quinolinecarboxylic acid (2) (Intermediate compound)

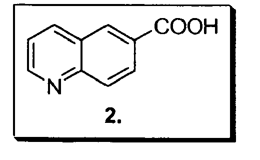

To a solution of 6-methylquinoline (100.0 mg, 0.70 mmol) in H₂O (1.0 mL) and H₂SO₄ (0.25 mL) chromium trioxide (272.0 mg, 2.72 mmol) was added in portions at 0°C and refluxing for twenty-four hours. The crystalline precipitate of the hydrosulphate which separated upon cooling was removed by filtration, dissolved in 10% sodium hydroxide water solution and, after wash with hexane, was reprecipitated with acetic acid to give 85.0 mg of title compound (70% yield) that was used in the following step without further purification. ¹H NMR, 300 MHz, (DMSO-*_{d6}*) δ 7.61 (dd, 1H, *J₁* = 8.3 Hz, *J₂* = 4.2 Hz), 8.08 (d, 1H, *J* = 8.8 Hz), 8.20 (dd, 1H, *J₁* = 8.8 Hz, *J₂* = 1.7 Hz), 8.56 (d, 1H, *J* = 8.2 Hz), 8.67 (m, 1H), 9.00 (dd, 1H, *J₁* = 4.1 Hz, *J₂* = 1.5 Hz), 13.20 (br s, 1H); ¹³C NMR, 300 MHz, (DMSO-*_{d6}*) 122.9, 127.9, 129.2, 129.5, 130.0, 131.7, 138.2, 150.0, 153.4, 167.7; ESI-MS *m*/*z* 196 [M+Na]⁺, 174 [M+H]⁺. Anal. (C₁₀H₇NO₂) C, H, N.

### N-(4-Hydroxybutyl)quinoline-6-carboxamide (3) (Intermediate compound)

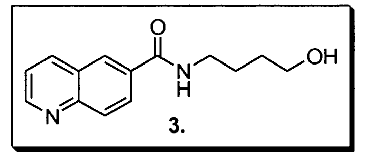

To a solution of 6-quinolinecarboxylic acid **(2)** (200.0 mg, 1.16 mmol) in dry dichloromethane (20.0 mL), triethylamine (162.0 µL, 1.16 mmol), 1-hydroxybenzotriazole hydrate (171.0 mg, 1.27 mmol) and *N*-(3-dimethylaminopropyl)-*N*'-ethylcarbodiimide hydrochloride (243.0 mg, 1.27 mmol) were added at 0°C under argon atmosphere; the suspension was warmed to room temperature and stirred for 1 h. Then 4-amino-1-butanol (117.0 µL, 1.27 mmol) was added and the mixture was stirred overnight at room temperature. The resulting suspension was evaporated and the crude product was purified by means of flash chromatography (10% methanol in chloroform) to give 275.0 mg of **3** as white solid (97% yield). Mp (methanol) 121-122°C; ¹H NMR, 300 MHz, (CDCl₃) δ 1.67-1.84 (m, 4H), 2.13 (br s, 1H); 3.48-3.59 (m, 2H), 3.76 (m, 2H), 7.02 (br s, 1H), 7.43 (m, 1H), 8.01-8.12 (m, 2H), 8.20 (d, 1H, *J* = 8.5 Hz), 8.30 (m, 1H), 8.94 (m, 1H). ESI-MS *m*/*z* 511 [2M+Na]⁺, 267 [M+Na]⁺, 245 [M+H]⁺. Anal. (C₁₄H₁₆N₂O₂) C, H, N.

### N-(4-Bromobutyl)quinoline-6-carboxamide (4) (Intermediate compound)

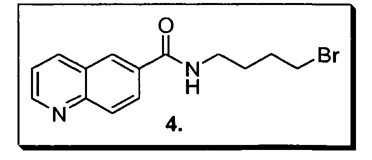

To a solution of *N*-(4-hydroxybutyl)quinoline-6-carboxamide **(5)** (500.0 mg, 2.05 mmol) in dry acetonitrile (30.0 mL), triphenylphosphine (808.0 mg, 3.08 mmol) and carbon tetrabromide (1021.0 mg, 3.08 mmol) were added under vigorous stirring at room temperature. After 2 h the mixture was quenched with 15% NaOH and extracted with EtOAc (3 x 10 mL). The organic layers were dried and evaporated. The residue was chromatographed (10% methanol in chloroform) to give 480.0 mg of **4** (75% yield) as yellow solid. ¹H NMR, 300 MHz, (CDCl₃) δ 1.66 (m, 2H), 1.77 (m, 2H), 3.26 (m, 2H), 3.36 (m, 2H), 7.22 (dd, 1H, *J₁* = 8.2 Hz, *J₂* = 4.4 Hz), 7.79 (br s, 1H), 7.88 (m, 2H), 7.97 (dd, 1H, *J₁* = 8.9 Hz, *J₂* = 1.9 Hz), 8.17 (d, 1H, *J* = 1.5 Hz), 8.75 (dd, 1H, *J₁*, = 4.3 Hz, *J₂* = 1.6 Hz); ESI-MS *m*/*z* 637 [2M+Na]⁺, 330 [M+Na]⁺, 308 [M+H]⁺. Anal. (C₁₄H₁₅BrN₂O) C, H, N.

### tert-Butyl 4-(2,3-difluorophenyl)piperazine-1-carboxylate (7) (Intermediate compound)

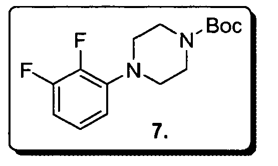

In a sealed tube, 1-bromo-2,3-difluorobenzene (517 mg, 2.68 mmol) Pd₂(dba)₂ (2%), BINAP (4%), and sodium *t*-butoxide (386.4 mg, 4.02 mmol) were added to *N*-Boc-piperazine (500 mg, 2.68 mmol) and the solids were dissolved in dry toluene (5 mL). The mixture was stirred at 70°C for 90 min., filtered over Celite®, washing with etylacetate and the organic layer was evaporated under reduced pressure. The crude was purified by flash chromatography (40% etylacetate in hexane) to give **7** as pail yellow solid (95% yield): ¹H NMR, 300MHz, (CDCl₃) δ 1.38 (s, 9H), 2.91 (m, 4H),3.48 (m, 4H), 6.55 (m, 1H), 6.66 (m, 1H), 6.83 (m, 1H). ESI-MS *m*/*z* 321 [M+Na⁺], 221, 199. Anal (C₁₅H₂₀F₂N₂O₂) C, H, N.

### 1-(2,3-Difluorophenyl)piperazine trifluoroacetate (8) (Intermediate compound)

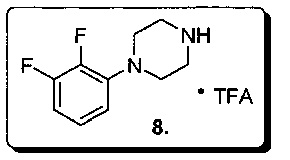

Trifluoroacetic acid (4 mL) was added to **7,** cooling in ice bath, and the mixture was stirred for 60 min. at room temperature. The crude was concentrated and washed with diethylether till the solid became colorless.

### N-(4-(4-Phenylpiperazin-1-yl)butyl)quinoline-6-carboxamide (Compound 5₁)

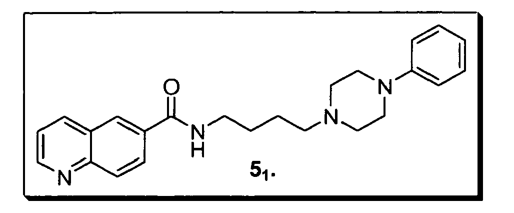

To a stirred solution of *N*-(4-bromobutyl)quinoline-6-carboxamide **(4)** (480.0 mg, 1.56 mmol) in dry acetonitrile (30.0 mL) under argon, 1-phenylpiperazine (238.0 µL, 1.56 mmol) and triethylamine (240.0 µL, 1.72 mmol) were added and the solution was refluxed overnight under stirring. The solvent was removed and the crude product was chromatographed (10% methanol in chloroform) to give 390.0 mg of **5₁** (65% yield) as white solid: mp (methanol) 151-152°C; ¹H NMR, 300 MHz, (CDCl₃) δ 1.65-1.75 (m, 4H), 2.45 (m, 2H), 2.58 (m, 4H), 3.14 (m, 4H), 3.54 (m, 2H), 6.84 (m, 3H), 7.07 (br s, 1H), 7.23 (m, 2H), 7.41 (m, 1H), 8.03 (m, 1H), 8.13 (m, 2H), 8.26 (m, 1H), 8.95 (m, 1H); ¹³C NMR (CDCl₃); 24.7, 27.6, 40.4, 49.2, 53.4, 58.1, 116.2, 120.0, 122.1, 127.5, 127.8, 129.3, 130.1, 133.1, 137.1, 149.5, 151.4 (2C^{IV}), 125.1, 167.3; ESI-MS *m*/*z* 411 [M+Na]⁺, 389 [M+H]⁺. Anal. (C₂₄H₂₈N₄O) C, H, N.

### Quinoline-6-carboxylic acid {4-[4-(2,3-difluoro-phenyl)-piperazin-1-yl]-butyl}-amide (Compound 5₂)

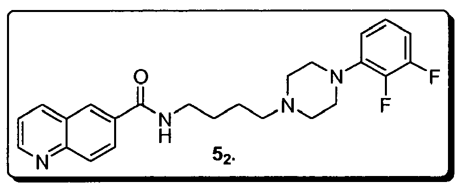

To a stirred solution of *N*-(4-bromobutyl)quinoline-6-carboxamide **(4)** (120.0 mg, 0.39 mmol) in dry acetonitrile (10.0 mL) under argon, 1-(2,3-difluorophenyl)piperazine trifluoroacetate **(8)** (181 mg, 0.585 mmol) and triethylamine (109 µL, 0.78 mmol) were added and the solution was refluxed overnight under stirring. The solvent was removed and the crude product was chromatographed (10% methanol in chloroform) to give 100.0 mg of **5₂** (60% yield) as amorphous solid: ¹H NMR, 300 MHz, (CDCl₃) δ 1.72 (m, 4H), 2.47 (t, 2H, *J* = 6.9 Hz), 2.61 (m, 4H), 3.06 (m, 4H), 3.54 (q, 2H, *J* = 6.1 Hz), 6.55 (t, 1H, *J* = 7.8 Hz), 6.76 (m, 1H), 6.92 (m, 2H), 7.45 (m, 1H), 8.03 (m, 1H), 8.15 (m, 1H), 8.25 (m, 1H), 8.29 (m, 1H), 8.97 (m, 1H). ESI-MS *m*/*z* 447 [M+Na]⁺, 425 [M+H]⁺. Anal. (C₂₄H₂₆F₂N₄O) C, H, N.

### Example 2

### Biological Activity

### In vitro Binding Studies

The affinity of Compound 5 of the invention for the dopamine and serotonin receptor subtypes was determined using standard receptor binding assays accomplished by MDS Pharma Services using the assay conditions specified below.

From these determinations the Compound 5 of the invention was found to be selective for dopamine D3 and having a Ki in the subnanomolar range.

### Dopamin D3 (MDS Catalog No. 219800)

Human recombinant CHO cells
Ligand = 0.7 nM [³H]-Spiperone
Non-specific = 25 µM S(-)-Sulpiride

### Serotonin (5-Hydroxytryptamine) 5-HT_{1A} (MDS Catalog No. 271110)

Human recombinant (CHO cells)
Ligand = 1.5 nM [³H] 8-OH-DPAT
Non-specific = 10 µM Metergoline

### Serotonin (5-Hydroxytryptamine) 5-HT_{2A} (MDS Catalog No. 271650)

Human recombinant (CHO cells)
Ligand = 0.5 nM [³H] Ketanserin
Non-specific = 1 µM Mianserin

## Claims

1. An aryl piperazine derivative represented by Formula I 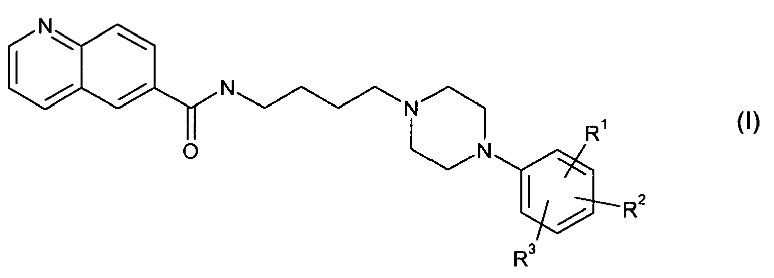 an enantiomer thereof or a mixture of its enantiomers, or a pharmaceutically acceptable salt thereof, wherein R¹, R² and R³, independently of each other, represent hydrogen, methyl, hydroxy, methoxy, halo, trifluoromethyl, cyano or carboxy.

2. The aryl piperazine derivative of claim 1, or a pharmaceutically acceptable salt thereof, wherein R¹, R² and R³, independently of each other, represent hydrogen, methyl, hydroxy, methoxy, halo or trifluoromethyl.

3. The aryl piperazine derivative of claims 1, or a pharmaceutically acceptable salt thereof, wherein R¹, R² and R³, independently of each other, represent hydrogen, halo, hydroxy or trifluoromethyl.

4. The aryl piperazine derivative of claim 1, or a pharmaceutically acceptable salt thereof, wherein R¹, R² and R³, independently of each other, represent hydrogen, fluoro, chloro, bromo or trifluoromethyl.

5. The aryl piperazine derivative of claim 1, or a pharmaceutically acceptable salt thereof, wherein
one of R¹, R² and R³, represents hydrogen or hydroxy; and
the two others of R¹, R² and R³, independently of each other, represent methyl, methoxy, halo, trifluoromethyl, cyano or carboxy.

6. The aryl piperazine derivative of claim 1, or a pharmaceutically acceptable salt thereof, wherein
two of R¹, R² and R³, represents hydrogen; and
the last one of R¹, R² and R³ represent methyl, hydroxy, methoxy, halo, trifluoromethyl, cyano or carboxy.

7. The aryl piperazine derivative of claim 1, which is
*N*-(4-(4-Phenylpiperazin-1-yl)butyl)quinoline-6-carboxamide; or
Quinoline-6-carboxylic acid {4-[4-(2,3-difluoro-phenyl)-piperazin-1-yl]-butyl}-amide;
an enantiomer thereof or a mixture of its enantiomers, or a pharmaceutically acceptable salt thereof.

8. A pharmaceutical composition comprising a therapeutically effective amount of an aryl piperazine derivative of any one of claims 1-7, or a pharmaceutically- acceptable addition salt thereof, together with at least one pharmaceutically acceptable carrier or diluent.

9. The aryl piperazine derivative of any one of claims 1-7, or a pharmaceutically acceptable salt thereof, for use as a medicament.

10. Use of the aryl piperazine derivative of any one of claims 1-7, or a pharmaceutically acceptable salt thereof, for the manufacture of a pharmaceutical composition.

11. Use of the aryl piperazine derivative of any one of claims 1-7, or a pharmaceutically acceptable salt thereof, for the manufacture of a pharmaceutical composition for the treatment, prevention or alleviation of psychotic disorders, ind. scnizophrenia, depression, Parkinson's disease, Huntington's disease, movement disorders, dystonia, anxiety, restlessness, obsessive-compulsive disorders, mania, geriatric disorders, dementia, sexual dysfunction, musculo-skeletal pain symptoms, pain associated with fibromyalgia, sleep disorders, substance abuse or addiction, abuse liability and withdrawal symptoms in drug addicts, cocaine abuse or addiction.

12. The use according to claim 11, wherein the disease or a disorder or a condition is schizophrenia.

## Patentansprüche

1. Arylpiperazinderivat, das durch Formel I wiedergegeben ist 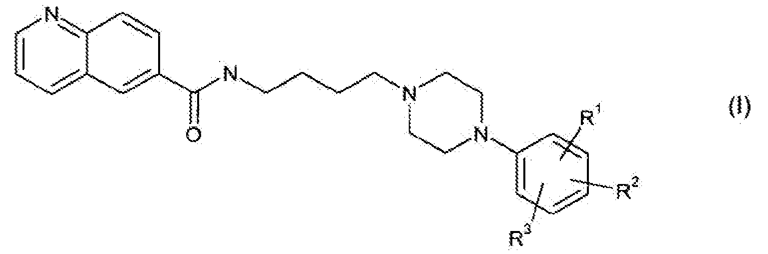 ein Enantiomer davon oder eine Mischung von seinen Enantiomeren, oder ein pharmazeutisch verträgliches Salz davon, wobei R¹, R² und R³, unabhängig voneinander, Wasserstoff, Methyl, Hydroxy, Methoxy, Halogen, Trifluormethyl, Cyano oder Carboxy bedeuten.

2. Arylpiperazinderivat nach Anspruch 1, oder ein pharmazeutisch verträgliches Salz davon, wobei R¹, R² und R³, unabhängig voneinander, Wasserstoff, Methyl, Hydroxy, Methoxy, Halogen oder Trifluormethyl bedeuten.

3. Arylpiperazinderivat nach Anspruch 1, oder ein pharmazeutisch verträgliches Salz davon, wobei R¹, R² und R³, unabhängig voneinander, Wasserstoff, Halogen, Hydroxy oder Trifluormethyl bedeuten.

4. Arylpiperazinderivat nach Anspruch 1, oder ein pharmazeutisch verträgliches Salz davon, wobei R¹, R² und R³, unabhängig voneinander, Wasserstoff, Fluor, Chlor, Brom oder Trifluormethyl bedeuten.

5. Arylpiperazinderivat nach Anspruch 1, oder ein pharmazeutisch verträgliches Salz davon, wobei
eines von R¹, R² und R³ Wasserstoff oder Hydroxy bedeutet; und
die beiden anderen von R¹, R² und R³, unabhängig voneinander, Methyl, Methoxy, Halogen, Trifluormethyl, Cyano oder Carboxy bedeuten.

6. Arylpiperazinderivat nach Anspruch 1, oder ein pharmazeutisch verträgliches Salz davon, wobei
zwei von R¹, R² und R³ Wasserstoff bedeuten; und
das letzte von R¹, R² und R³ Methyl, Hydroxy, Methoxy, Halogen, Trifluormethyl, Cyano oder Carboxy bedeutet.

7. Arylpiperazinderivat nach Anspruch 1, welches
*N*-(4-(4-Phenylpiperazin-1-yl)butyl)chinolin-6-carboxamid; oder
Chinolin-6-carbonsäure-{4-[4-(2,3-difluor-phenyl)-piperazin-1-yl]-butyl}-amid ist;
ein Enantiomer davon oder eine Mischung von seinen Enantiomeren, oder ein pharmazeutisch verträgliches Salz davon.

8. Pharmazeutische Zusammensetzung, umfassend eine therapeutisch wirksame Menge von einem Arylpiperazinderivat nach einem der Ansprüche 1-7, oder einem pharmazeutisch verträglichen Additionssalz davon, zusammen mit wenigstens einem pharmazeutisch verträglichen Träger oder Verdünnungsmittel.

9. Arylpiperazinderivat nach einem der Ansprüche 1-7, oder ein pharmazeutisch verträgliches Salz davon, zur Verwendung als ein Medikament.

10. Verwendung des Arylpiperazinderivats nach einem der Ansprüche 1-7, oder eines pharmazeutisch verträglichen Salzes davon, für die Herstellung einer pharmazeutischen Zusammensetzung.

11. Verwendung des Arylpiperazinderivats nach einem der Ansprüche 1-7, oder eines pharmazeutisch verträglichen Salzes davon, für die Herstellung einer pharmazeutischen Zusammensetzung für die Behandlung, Verhütung oder Linderung von psychotischen Störungen einschließlich Schizophrenie, Depression, Parkinson-Krankheit, Chorea Huntington, Bewegungsstörungen, Dystonie, Angst, Ruhelosigkeit, Zwangsstörungen, Manie, geriatrischen Störungen, Demenz, sexueller Dysfunktion, muskuloskelettalen Schmerzsymptomen, Schmerz in Zusammenhang mit Fibromyalgie, Schlafstörungen, Substanzmissbrauch oder -abhängigkeit, Missbrauchsanfälligkeit und Entzugssymptomen bei Drogenabhängigen, Kokainmissbrauch oder-abhängigkeit.

12. Verwendung nach Anspruch 11, wobei es sich bei der Krankheit oder einer Störung oder einem Zustand um Schizophrenie handelt.

## Revendications

1. Dérivé d'aryl pipérazine représenté par la formule I 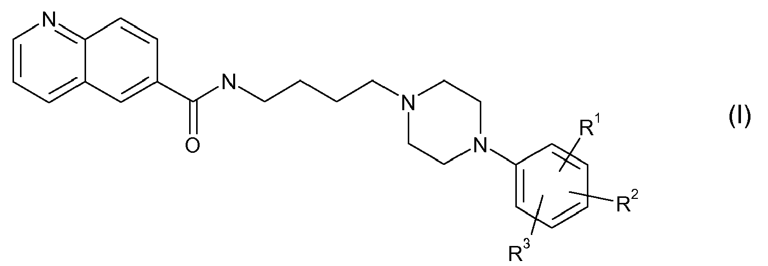 un énantiomère de celui-ci ou un mélange de ses énantiomères, ou un sel pharmaceutiquement acceptable de celui-ci, dans laquelle R¹, R² et R³, indépendamment les uns des autres, représentent un hydrogène, un méthyle, un hydroxy, un méthoxy, un halogéno, un trifluorométhyle, un cyano ou un carboxy.

2. Dérivé d'aryl pipérazine selon la revendication 1, ou un sel pharmaceutiquement acceptable de celui-ci, dans lequel R¹, R² et R³, indépendamment les uns des autres, représentent un hydrogène, un méthyle, un hydroxy, un méthoxy, un halogéno ou un trifluorométhyle.

3. Dérivé d'aryl pipérazine selon la revendication 1, ou un sel pharmaceutiquement acceptable de celui-ci, dans lequel R¹, R² et R³, indépendamment les uns des autres, représentent un hydrogène, un halogéno, un hydroxy ou un trifluorométhyle.

4. Dérivé d'aryl pipérazine selon la revendication 1, ou un sel pharmaceutiquement acceptable de celui-ci, dans lequel R¹, R² et R³, indépendamment les uns des autres, représentent un hydrogène, un fluoro, un chloro, un bromo ou un trifluorométhyle.

5. Dérivé d'aryl pipérazine selon la revendication 1, ou un sel pharmaceutiquement acceptable de celui-ci, dans lequel
l'un parmi R¹, R² et R³ représente un hydrogène ou un hydroxy ; et
les deux autres parmi R¹, R² et R³, indépendamment les uns des autres, représentent un méthyle, un méthoxy, un halogéno, un trifluorométhyle, un cyano ou un carboxy.

6. Dérivé d'aryl pipérazine selon la revendication 1, ou un sel pharmaceutiquement acceptable de celui-ci, dans lequel
deux parmi R¹, R² et R² représentent un hydrogène ; et
le dernier parmi R¹, R² et R³ représente un méthyle, un hydroxy, un méthoxy, un halogéno, un trifluorométhyle, un cyano ou un carboxy.

7. Dérivé d'aryl pipérazine selon la revendication 1, qui est
le *N*-(4-(4-phénylpipérazin-1-yl)butyl)quinoléine-6-carboxamide ; ou
l'amide {4-[4-(2,3-difluoro-phényl)-pipérazin-1-yl]-butylique} de l'acide quinoléine-6-carboxylique ;
un énantiomère de celui-ci ou un mélange de ses énantiomères, ou un sel pharmaceutiquement acceptable de celui-ci.

8. Composition pharmaceutique comprenant une quantité thérapeutiquement efficace d'un dérivé d'aryl pipérazine selon l'une quelconque des revendications 1 à 7, ou d'un sel d'addition pharmaceutiquement acceptable de celui-ci, conjointement avec au moins un support ou diluant pharmaceutiquement acceptable.

9. Dérivé d'aryl pipérazine selon l'une quelconque des revendications 1 à 7, ou un sel pharmaceutiquement acceptable de celui-ci, pour l'utilisation en tant que médicament.

10. Utilisation du dérivé d'aryl pipérazine selon l'une quelconque des revendications 1 à 7, ou d'un sel pharmaceutiquement acceptable de celui-ci, pour la fabrication d'une composition pharmaceutique.

11. Utilisation du dérivé d'aryl pipérazine selon l'une quelconque des revendications 1 à 7, ou d'un sel pharmaceutiquement acceptable de celui-ci, pour la fabrication d'une composition pharmaceutique pour le traitement, la prévention ou l'atténuation de troubles psychotiques incluant la schizophrénie, d'une dépression, d'une maladie de Parkinson, d'une maladie de Huntington, de troubles du mouvement, d'une dystonie, d'une anxiété, d'une agitation, de troubles obsessivo-compulsifs, d'une manie, de troubles gériatriques, d'une démence, d'un dysfonctionnement sexuel, de symptômes de douleur musculo-squelettique, d'une douleur associée à une fibromyalgie, de troubles du sommeil, d'une utilisation abusive d'une substance ou d'une toxicomanie, d'une tendance à une utilisation abusive et de symptômes de sevrage chez des toxicomanes, d'une utilisation abusive de cocaïne ou d'une cocaïnomanie.

12. Utilisation selon la revendication 11, dans laquelle la maladie ou un trouble ou une affection est une schizophrénie.
